# EUROPÄISCHE PATENTSCHRIFT

(11) **EP 1 290 438 B1**
(45) Veröffentlichungstag und Bekanntmachung des Hinweises auf die Patenterteilung: **29.09.2004**
(21) Anmeldenummer: 01951341.5
(22) Anmeldetag: 20.05.2001
(51) Int. Cl.: G01N 33/15, G01N 3/40

(54) **VERFAHREN UND VORRICHTUNG ZUM AUSRICHTEN UND VERSCHIEBEN EINES PRÜFLINGS, WIE TABLETTEN, PILLEN ODER TABLETS**
METHOD AND DEVICE FOR THE ALIGNMENT AND LOCATION OF A SAMPLE SUCH AS TABLETS, PILLS OR TABLETTES
PROCEDE ET DISPOSITIF POUR ORIENTER ET DEPLACER UNE EPROUVETTE, PAR EXEMPLE DES COMPRIMES, PILULES OU PASTILLES

(30) Priorität: 22.05.2000 DE 10024970
(43) Veröffentlichungstag der Anmeldung: 12.03.2003
(73) Patentinhaber: Kraemer, Thilo, 64291 Darmstadt (DE)
(72) Erfinder: KRAEMER, Norbert, 64291 Darmstadt (DE)
(74) Vertreter: Mierswa, Klaus, Dipl.-Ing.
(86) Internationale Anmeldenummer: PCT/DE2001/001903
(87) Internationale Veröffentlichungsnummer: WO 2001/090743

(56) Entgegenhaltungen:
- WO-A-85/03278
- WO-A-98/19945
- DE-A- 19 744 227
- US-A- 4 393 717
- US-A- 5 140 861
- US-A- 5 555 768

## Beschreibung

### Technisches Gebiet:

Die Erfindung betrifft ein Verfahren zum Ausrichten und Verschieben eines Prüflings, wie Tabletten, Pillen, Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozeßschrittes, wie Härtetest, mit dem Prüfling, unter Zuhilfenahme von zwei sich gegenüberstehenden und relativ zueinander beweglichen Backen, von denen der bewegliche Preßbacken den Prüfling auf einer Führungsbahn auf den feststehenden Gegenbacken bis zum mechanischen Kontakt des Prüflings mit dem Gegenbacken vorschiebt, um den weiteren Prozeßschritt mit dem Prüfling einzuleiten, gemäß dem Oberbegriff des Anspruchs 1. Ebenso betrifft die Erfindung eine Vorrichtung zur Durchführung des Verfahrens gemäß dem Oberbegriff des Anspruchs 6 oder des Anspruchs 12.

### Stand der Technik:

Im Rahmen der Qualitätskontrolle bei der Fertigung von Tabletten, Pillen oder Dragees, aber auch bei der Herstellung von Tablets in der Waschmittelindustrie, werden mechanisch-physikalische Eigenschaften derselben, wie z.B. Gewicht, Abmessungen, Zerfallszeit in einem Medium und Härte bestimmt. Dazu sind Tablettenprüfsysteme bekannt, welche jeweils eines Mehrzahl von Tabletten aus einem Produktionszyklus auf diese Eigenschaften hin untersuchen. Tabletten einer Charge werden aus einem Vorratsbehälter vereinzelt und z.B. mittels eines Transportsterns oder mittels eines Transportbandes von einer Meßstation zur nächsten befördert. Derartige Transportsterne besitzen peripher in der Regel 24 Kammern zur vereinzelten Aufnahme je eines Tablettenprüflings. Zuerst erfolgt ein Wiegevorgang, an den sich eine Messeinrichtung zur Ermittlungen der Abmessungen des Prüflings anschließt, auf die ein Härtetester folgt. Das Zusammenspiel der einzelnen Stationen sowie die Speicherung und Übermittlung der Messergebnisse übernimmt eine Software sowie eine zentrale Recheneinheit.

Die Härte eines Prüflings wird üblicherweise in einer fein auflösenden Kraftmeßdose gemessen, die einen Druckkolben und ein Gegenlager, nämlich feststehender Gegenbacken und beweglicher Preßbacken, aufweist. Der Prüfling wird in den Bereich zwischen Gegenbacken und Preßbacken auf eine Führungsbahn befördert, wobei der Prüfling vorzugsweise den Gegenbacken berührt. Der Preßbacken wird nun mittels eines Schrittmotors gegen den Gegenbacken und den vor diesem liegenden Prüfling gefahren. Die vom Preßbacken mit jedem Schritt des Motors ausgeübte Kraft wird gemessen und aufgezeichnet, die konstant und sehr klein ist, solange der Gegenbacken den Prüfling nicht berührt oder dieser ohne den Gegendruck des Gegenbackens über die Führungsbahn geschoben wird. Wenn der Preßbacken den Prüfling gegen den Gegenbacken drückt, steigt die von ihm ausgeübte Kraft mit jedem Schritt des Schrittmotors so lange an, bis der Prüfling zerbricht. Die dazu aufgewendete Kraft wird aufgezeichnet und dient als Maß für die Härte des Prüflings. Das plötzliche Abfallen der vom Preßbacken aufgewendeten Kraft beim Zerbrechen des Prüflings dient als Abbruchbedingung für das Beenden der Messung. Der Preßbacken wird in seine Ausgangsposition zurückgefahren, und der nächste Prüfling kann geprüft werden.

Eine solche Vorrichtung zur Durchführung eines Härtetests ist durch die WO 98/53298 bekannt geworden ist, die einen Prüftisch zur Aufnahme des Prüflings sowie einen linear verfahrbaren Druckkolben und ein Gegenlager aufweist, die oberhalb des Prüftisches angeordnet und gegeneinander verfahrbar sind. Der Prüfling befindet sich zwischen Druckkolben und Gegenlager, wobei die beim Gegeneinanderdrücken der beiden aufgewendete Kraft oder eine dazu proportionale Größe mittels einer Kraftmeßvorrichtung meßbar und die Härte des Prüflings daraus bestimmt wird.

Prüflinge, welche jedoch wenigstens eine bombierte oder gewölbte Hauptoberfläche aufweisen, beispielsweise linsenförmig gestaltet sind, verkippen beim Verschieben mittels des Preßbackens und gelangen beim weiteren Vorschieben dergestalt verkippt zwischen Preßbacken und Gegenbacken, so dass in verkipptem Zustand des Prüflings kein aussagekräftiger Härtetest durchgeführt werden kann; u.U. stellt sich der Prüfling zwischen den Backen sogar senkrecht. Ein Prüfling muß aber zur Bestimmung seines Durchmessers wie auch zur Durchführung eines Härtetests waagrecht auf der Führungsbahn aufliegen, denn nur die in dieser Lage gemessene Kraft beim Härtetest ist aussagekräftig und zum Beispiel pharmazeutisch zugelassen.

Aus der DE 196 54 612 C2 ist ein Verfahren und eine Vorrichtung zur Kontrolle von Tablettenparametern bekannt geworden, welche eine Rütteleinrichtung aufweist, um die Tablette in eine definierte und stabile Schwerpunktlage zur Messung der Tablettenhöhe zu bringen. Dadurch soll gewährleistet sein, dass die Tablette unabhängig von ihrer Form in der Höhe bzw. ihrer dicke nach den bekannten Verfahren gemessen werden kann. Die Rüttelvorrichtung besteht aus zwei waagrecht übereinander angeordneten und gekoppelten Platten, wobei auf der oberen Platte die Tablette zur Durchführung der Messungen positioniert wird und die untere Platte die Rüttelbewegungen ausführt, welche auf die obere Platte übertragen werden.

Die US 4,393,717 beschreibt ein Tablettenprüfgerät, welches zum Tablettentransport eine mit einem Vibrator gekoppelte V-förmige und geneigte Rinne aufweist zum vereinzelnden Transport der Tabletten durch die Rinne.

Durch die DE 35 41 672 A1 ist eine Vorrichtung an Verpackungsmaschinen zum geordneten Zuführen und Ablegen zu verpackender Kleinteile, wie Tabletten, Kapseln, Dragees, bekannt geworden, welche zwei relativ zueinander bewegliche, sich kreuzende Schieber aufweist. Den Schiebern wird zeitweise eine Rüttelbewegung aufgeprägt, um die Ausrichtung und Aufnahme der Kleinteile in entsprechend geformte Aussparungen in den Schiebern zu erleichtern. Der untere der beiden Schieber kann eine Vorschubbewegung ausführen, um die Kleinteile in passende Positionen zwecks Verpackung zu bringen.

### Technische Aufgabe:

Der Erfindung liegt die Aufgabe zugrunde, ein Verfahren und eine Vorrichtung der genannten Gattung zu schaffen, mit welchem auch die Messung des Durchmessers eines Prüflings, der wenigstens eine bombierte oder gewölbte Hauptoberfläche aufweist, beispielsweise linsenförmig gestaltet ist, sowie die Durchführung eines Härtetests am Prüfling sowie das Verschieben bzw. Überschieben des Prüflings möglich ist, wobei das Verkippen des Prüflings ausgeschlossen sein soll.

Diese Aufgabe wird durch die Merkmale der Ansprüche 1, 6 bzw. 12 gelöst.

### Offenbarung der Erfindung sowie deren Vorteile:

Die verfahrensmäßige Lösung der Aufgabe besteht erfindungsgemäß darin, dass unter Mitnahme des Prüflings die Vorschubbewegung des beweglichen Preßbackens in Richtung des feststehenden Gegenbackens fortlaufend unterbrochen und der Preßbacken zurückgezogen und vorgeschoben wird, wobei die Vorschub- und Rückschubbewegung des Preßbackens klein ist in Bezug auf seinen gesamten Vorschubweg bezüglich des Gegenbackens, insgesamt jedoch der Preßbacken auf den Gegenbacken unter Mitnahme des Prüflings um jeweils die kleine Vorschubstrecke und Lösen von dem Prüfling mittels Rückschub zufährt, so dass der Prüfung in seine Ruhelage zurückschwingt und danach der Preßbacken wieder zum Schub in Richtung des Gegenbackens ansetzt, bis der Prüfling den Gegenbacken erreicht hat oder der feststehende Backen und/oder die Führungsbahn wird während der Vorschubbewegung des beweglichen Preßbackens in Richtung des feststehenden Gegenbackens in Schwingungen versetzt, welche sich dem Prüfling aufprägen, so dass derselbe während der Vorschubbewegung des Preßbackens auch eine Kippschwingung und/oder Vibrationsschwingung durchführt, die den Prüfling fortwährend in die Ruhelage zurück schwingen oder um dieselbe schwingen lässt, bis der Prüfling den Gegenbacken erreicht hat und die Schwingung des Prüflings durch den feststehenden Gegenbacken angehalten wird. Die Vor- und Zurückbewegung des beweglichen Preßbackens in Richtung des feststehenden Gegenbackens wird so lange ausgeführt, bis der Prüfling den feststehenden Gegenbacken gerade berührt. Nunmehr kann zuerst der Durchmesser des Prüflings bestimmt werden, anschließend kann zum Beispiel der Härtetest durchgeführt und der Prüfling zerbrochen werden.

Das erfindungsgemäße Verfahren besitzt den hervorstechenden Vorteil, dass nunmehr auch die Messung des Durchmessers sowie die Durchführung eines Härtetests an einem Prüfling möglich ist, der wenigstens eine bombierte oder gewölbte Hauptoberfläche aufweist, beispielsweise linsenförmig gestaltet ist. Das Arbeitsverfahren bewirkt, dass der Prüfling spätestens bei der Anlage am Gegenbacken zur Bestimmung des Durchmessers oder zur Durchführung des Härtetests waagrecht liegt; ein Verkippen wenigstens zu diesem Zeitpunkt ist ausgeschlossen. Prinzipiell kann die waagrechte Ausrichtung des Prüflings durch eine Hin- und Herbewegung des Preßbackens oder durch eine Vibration des Gegenbackens und/oder der Führungsbahn erfolgen.

In weiterer vorteilhafter Ausgestaltung der Erfindung schiebt der bewegliche Preßbacken den Prüfling vor sich her und verkippt dabei den Prüfling gegebenenfalls, bis derselbe an dem feststehenden Gegenbacken anfährt, wonach der bewegliche Preßbacken zurückgezogen wird, bis der Prüfling in seine Ruhelage geschwungen ist und anschließend der Preßbacken wiederum in Richtung des feststehenden Gegenbackens zur Einleitung des Härtetests fährt.

Auch kann das Andocken des Prüflings am feststehenden Gegenbacken mittels einer Videokamera überwacht werden, die beim Andocken ein Signal abgibt, welches zur Steuerung des Elektromotors zum Antrieb des beweglichen Preßbackens dient. In weiterer verfahrensmäßiger Ausgestaltung führt der bewegliche Preßbacken während seiner Vorschubbewegung in Richtung des feststehenden Gegenbackens Vibrationen in Richtung seiner Längsachse hin und zurück bezüglich des Gegenbackens aus, welche der Vorschubbewegung des Preßbackens überlagert werden.

Eine gattungsgemäße Vorrichtung zur Durchführung des Verfahrens besteht darin, dass der Preßbacken unter fortlaufender Unterbrechung seiner Vorschubbewegung kleine Vorschub- und Rückschubstrecken in Bezug auf seinen gesamten Vorschubweg vor und zurück bezüglich des Gegenbackens verfahrbar ist, insgesamt jedoch auf den Gegenbacken unter Mitnahme des Prüflings um jeweils die kleine Vorschubstrecke und Lösen von dem Prüfling mittels Rückschub, bis der Prüfling den Gegenbacken erreicht, zuzufahren imstande ist oder dass der feststehende Backen und/oder die Führungsbahn während der Vorschubbewegung des beweglichen Preßbackens in Richtung auf den feststehenden Gegenbacken um eine Ruhelage mittels eines Schwingungserzeugers in Schwingungen versetzbar ist, welche sich dem Prüfung aufprägen, so dass derselbe während der Vorschubbewegung auch eine Kippschwingung bzw. Vibrationsschwingung durchzuführen imstande ist. Damit sind prinzipiell die beiden Ausgestaltungen möglich, dass nämlich der Preßbacken hin und zurückfährt, jedoch im Sinne eines der Vorschubbewegung des Preßbackens überlagerten Hin- und Zurückfahrens, oder der Gegenbacken und/oder die Führungsbahn vibrieren.

Der bewegliche Preßbacken sowie eine daran befestigte Kraftmeßdose sind zur Bildung eines Härtetesters auf einem Schlitten angeordnet, welcher auf einer feststehenden Führungsschiene verfahrbar ist und welcher mittels eines elektrischen Schrittmotors sowie eines Antriebsvorgeleges um kleine Vorschub- und Rückschubstrecken in Bezug auf seinen gesamten Vorschubweg vor und zurück bezüglich des Gegenbackens verfahrbar ist.

Oberhalb des feststehenden Gegenbackens ist eine Videokamera zum Überwachen des Andockens des Prüflings an dem Gegenbacken angeordnet, die ein elektrisches Signal erzeugt, das zur Beeinflussung des Elektromotors dient.

Das Verfahren und die Vorrichtung zum Ausrichten und Verschieben eines Prüflings, wie Tabletten, Pillen, Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozeßschrittes, wie Härtetest oder Überschub, mit dem Prüfling, mit einem beweglichen Schubbacken zur Bewegung des Prüflings auf einer Führungsbahn zur Einleitung des weiteren Prozeßschritts mit dem Prüfling, kann auch losgelöst von einem Härtetestvorgang angewendet werden und hierfür ausgestaltet sein, zum Beispiel, wenn ein derartig geformter Prüfling waagrecht überschoben werden soll. In diesem Fall ist ein Schubbacken vorhanden, der unter fortlaufender Unterbrechung seiner Vorschubbewegung um kleine Vorschub- und Rückschubstrecken in Bezug auf seinen gesamten Vorschubweg vor und zurück verfahrbar ist, insgesamt jedoch unter Mitnahme des Prüflings auf der Führungsbahn um jeweils die kleine Vorschubstrecke und Lösen von dem Prüfling mittels Rückschub, bis der Prüfling den gewünschten Ort erreicht, zu verfahren imstande. Oder die Führungsbahn ist während der Vorschubbewegung des beweglichen Schubbackens um eine Ruhelage mittels eines Schwingungserzeugers in Schwingungen versetzbar, welche sich dem Prüfling aufprägen, so dass derselbe während der Vorschubbewegung des Schubbackens auch eine Kipp- und/oder Vibrationsschwingung durchzuführen imstande ist und die tanzende Bewegung des Prüflings erst durch den feststehenden Gegenbacken angehalten wird.

Kurzbezeichnung der Zeichnung, in der zeigen:
- Figur 1: eine schematische Gesamtansicht einer Härtetesteinrichtung
- Figur 2: eine Draufsicht auf Figur 1
- Figur 3 a bis f: die Bewegung des beweglichen Preßbackens in Bezug auf den feststehenden Gegenbacken
- Figuren 4 und 5: je eine Ansicht einer Härtetesteinrichtung mit einem Rüttler, welcher den Gegenbacken und oder die Führungsbahn, auf der sich der Prüfling befindet, in Vibrationen versetzt.

### Bevorzugtes Ausführungsbeispiel der Erfindung:

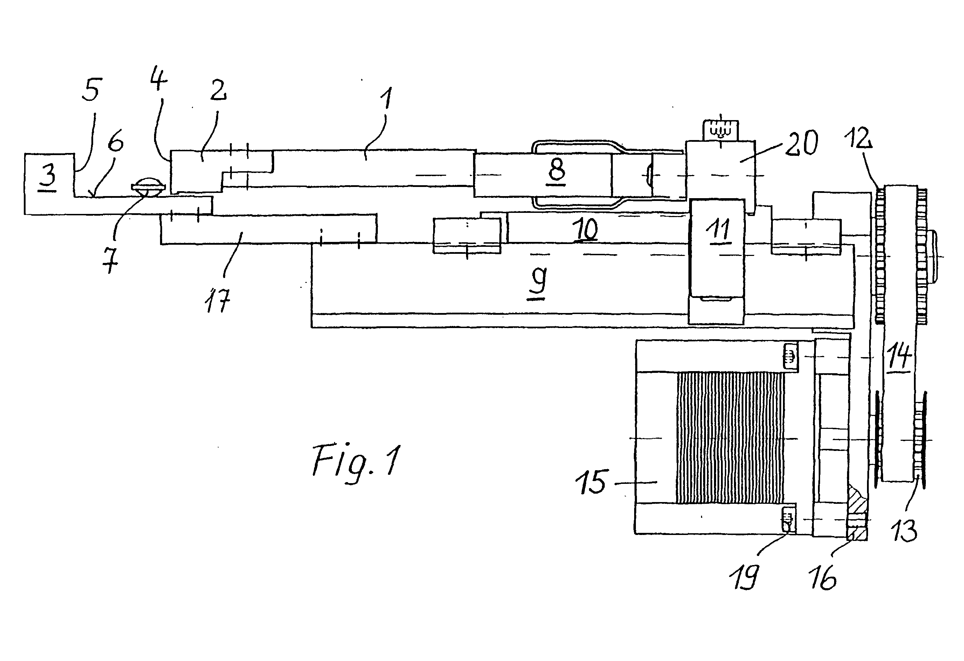

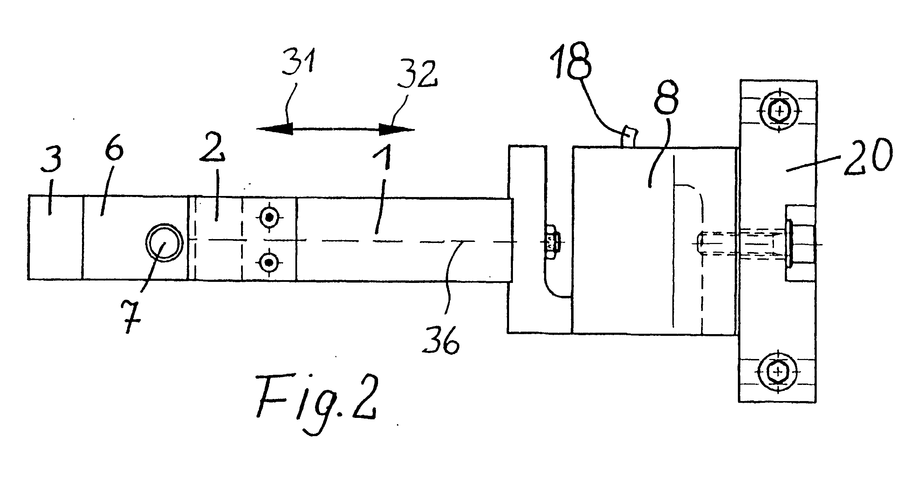

Gemäß den Figuren 1 und 2 ist als Beispiel eine Härtetesteinrichtung für Prüflinge, wie Tabletten, Pillen, Dragees oder Tablets, gezeigt, bestehend aus einem Stößel 1, der an seinem vorderen Ende einen Preßbacken 2 trägt, welcher eine senkrecht stehende Schubfläche 4 besitzt. Der Stößel ist an seinem hinteren Ende mit einer Kraftmeßdose 8, die eine elektrische Anschlußleitung 18 aufweist, verbunden, wobei die Kraftmeßdose 8 gemäß Figur 2 an einer Traverse 20 befestigt ist. Der Stößel 1 mitsamt der Kraftmeßdose 8 ist auf einen Schlitten 10 montiert, der längs in Richtung der Längsachse 36 des Stößels 1 auf einer fest angeordneten Führungsschiene 9 verfahrbar ist. Die Führungsschiene 9 trägt des Weiteren in ihrem vorderen Bereich ein Halteteil 17, an welchem eine Führungsbahn 6 sowie ein Gegenbacken 3 mit einer senkrechten Stirnwand 5 befestigt sind. Damit sind Gegenbacken 3, Führungsbahn 6, Halteteil 17 und Führungsschiene 9 gegenüber dem Schlitten 10 und somit dem Stößel 1 in Ruhe.

Ein Elektromotor 15, der vorzugsweise ein Schrittmotor ist, ist in geeigneter Weise mittels einer Platte 16 und Schrauben 19 an der Führungsschiene 9 befestigt und treibt über ein Zahnrad 13 sowie einen Zahnriemen 14 ein weiteres Zahnrad 12 an. Diese Zahnrad 12 ist über einen Mitnehmer 11 mit dem Schlitten 10 bzw. der Traverse 20 verbunden; über den Mitnehmer 11 wird bei Drehung des Zahnradvorgeleges vor oder zurück der Schlitten 10 vor oder zurück auf der Führungsschiene 9 bewegt. Auf diese Weise bewegt sich der Preßbacken 2 über der Führungsbahn 6 auf den Gegenbacken 3 zu oder von diesem weg.

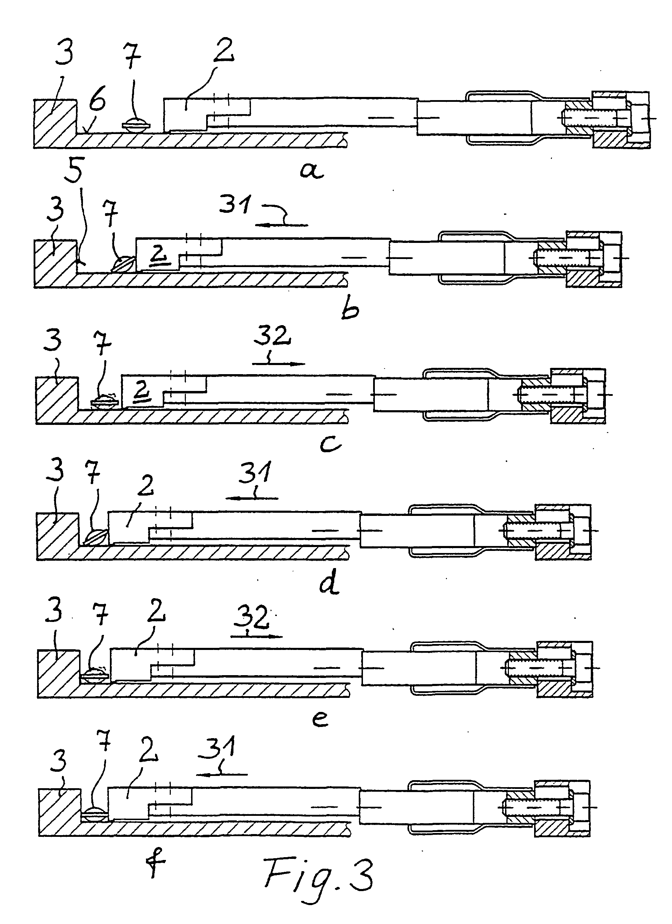

In Figur 3 a bis f ist ein linsenförmiger Prüfling 7 auf der Führungsbahn 6 zwischen dem feststehenden Gegenbacken 3 und dem beweglichen Preßbacken 2 gezeigt; der Prüfling 7 liegt in Figur 3a waagrecht in seiner Ruhelage. Nunmehr fährt gemäß Figur 3a der Preßbacken 2 in Richtung des Bewegungspfeils 31 auf den Gegenbacken 3 zu, nimmt dabei den Prüfling 7 mit und verkippt ihn. Daraufhin fährt der Preßbacken 2 gemäß Figur 3c in Richtung des Bewegungspfeils 32 um eine kleine Wegstrecke zurück, so dass der Prüfling 7 wieder in seine Ruhelage zurückschwingen kann. Nunmehr fährt der Preßbacken 2 erneut in Richtung des Bewegungspfeils 31 gemäß Figur 3d an den Prüfling an, verschiebt und verkippt ihn wiederum, wobei der Preßbacken 2 nahe des gegenüberliegenden Gegenbackens 3 fährt. Gemäß Figur 3e fährt der Preßbacken wiederum eine kleine Wegstrecke - in Bezug auf den gesamten Verfahrweg des Preßbackens 2 - zurück, so dass der Prüfling 7 wiederum in seine Ruhelage zurückschwingen kann und dabei entweder die Stirnwand 5 des Gegenbackens 3 schon berührt oder fast berührt. Die kleine Wegstrecke, um die der Preßbachen 2 zurückfahren muß, damit der Prüfling 7 wieder seine Ruhelage einnehmen kann, ist durch den Kosinus des Stellwinkels des Prüflings 7 gegeben. Sobald der Prüfling seine in Figur 3e gezeigte waagrechte Lage eingenommen hat, fährt der Preßbacken 2 gemäß der Figur 3f wieder in Richtung des Bewegungspfeils 31 auf den Gegenbacken 3 zu, wobei mit der bloßen Klemmung des Prüflings zwischen den Backen der Durchmesser des Prüflings 7 (als Funktion des Kraftanstiegs) gemessen wird. Bei weiterer Vorschubbewegung des Preßbackens 2 gegenüber dem Gegenbacken 3 wird der Härtetest des Prüflings 7 eingeleitet bis zu dessen Zerbersten.

Somit wird unter Mitnahme des Prüflings 7 die Vorschubbewegung des beweglichen Preßbackens 2 in Richtung des feststehenden Gegenbackens 3 fortlaufend unterbrochen und der Preßbacken 2 zurückgezogen und vorgeschoben, wobei die Vorschub- und Rückschubbewegung des Preßbackens 2 klein ist in Bezug auf seinen gesamten Vorschubweg bezüglich des Gegenbackens 3; insgesamt jedoch fährt der Preßbacken 2 auf den Gegenbacken 3 unter Mitnahme des Prüflings 7 um jeweils die kleine Vorschubstrecke zu und jeweils unter Loslösen von dem Prüfling mittels Rückschub zurück, so dass der Prüfling in seine Ruhelage zurückschwingt und danach der Preßbacken 2 wieder zum Schub in Richtung des Gegenbackens 3,21 ansetzt, bis der Prüfling 7 den Gegenbacken ,21 erreicht hat. Diese Vor- und Zurückbewegung des beweglichen Preßbackens 2 in Richtung des feststehenden Gegenbackens 3 wird so lange ausgeführt, bis der Prüfling 7 den feststehenden Gegenbacken 3 gerade berührt.

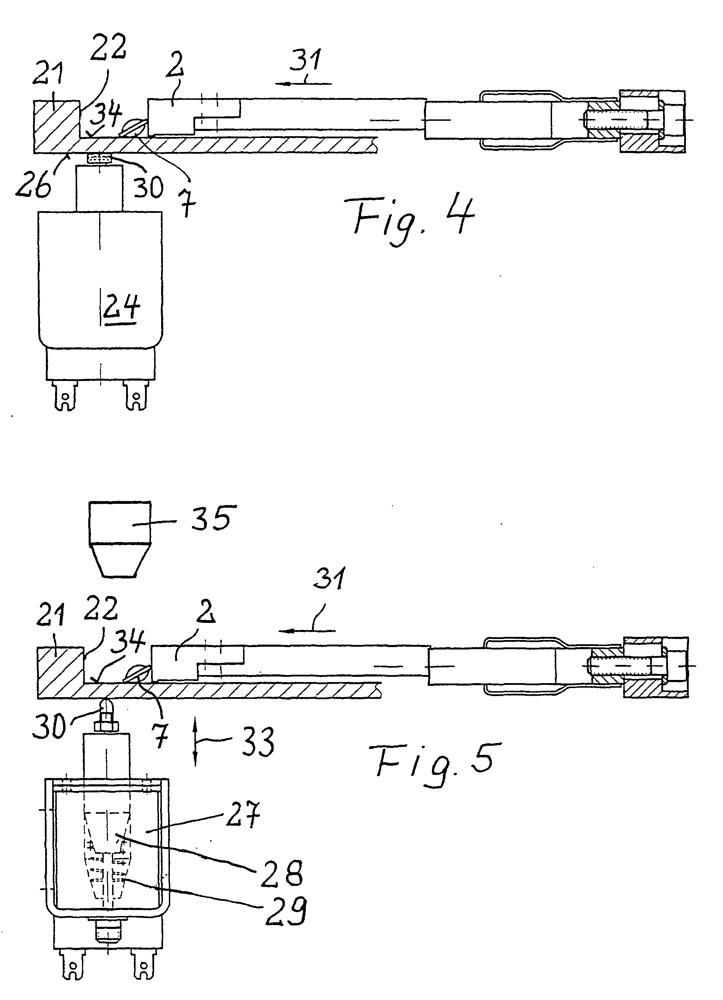

In den Figuren 4 und 5 sind zwei Ausführungsbeispiele gezeigt, in denen der Preßbacken während seine Vorschubs keine überlagerten Vor- und Zurückbewegungen zur waagrechten Ausrichtung des Prüflings 7 ausführt. Sondern der feststehende Backen 21 bzw. die Führungsbahn 34 wird während der Vorschubbewegung des beweglichen Preßbackens 2 in Richtung des feststehenden Gegenbackens 21 mittels eines Rüttlers 24, 27 oder Vibrators in Schwingungen versetzt, welche sich dem Prüfling 7 aufprägen, so dass derselbe während der Vorschubbewegung des Preßbackens 2 wohl anfangs im Augenblick des Beginns der Vorschubbewegung verkippt wird, jedoch auch eine Kippschwingung bzw. Vibrationsschwingung bzw. tanzende Bewegung durchführt, die den Prüfung 7 fortwährend in seine Ruhelage zurückschwingen oder um dieselbe schwingen lässt, bis der Prüfling 7 den Gegenbacken 21 erreicht hat und die Schwingung des Prüflings 7 durch den feststehenden Gegenbacken 21 angehalten wird.

Der Rüttler 24, 27 besitzt jeweils einen Stößel 30, welcher unterhalb der Führungsbahn 34 nahe des Gegenbackens 21 angreift und seine Schwingungen auf den Gegenbacken 21 und/oder auf die Führungsbahn 34 überträgt. Dadurch führt der Prüfling 7 eine tanzende Bewegung aus, bis er an der senkrechten Stirnwand 22 des Gegenbackens anstößt.

Der in Figur 5 gezeigte Rüttler ist ein elektromagnetischer Rüttler 27, der zur Erzeugung der Vibration einen Elektromagneten 28 aufweist, welcher mittels einer Rückstellfeder 29 rückgestellt wird. Oberhalb der Backen 2, 21 und insbesondere im Bereich der Gegenbacke 21 kann eine Videokamera 35 angeordnet sein zur optischen Überwachung des Andockens oder Anfahrens des Prüflings 7 an den Gegenbacken. Ein gewonnenes optisches Signal wird in ein Ausgangssignal gewandelt, welches zur Ansteuerung und Beeinflussung des Elektromotors 15 (Figur 1) verwendet werden kann.

### Gewerbliche Anwendbarkeit:

Die Erfindung ist insbesondere für den Einsatz in Tablettenprüfstationen geeignet, in welchen linsenförmige oder bombierte oder oval geformte Tabletten oder Pillen oder Dragees oder Tablets hinsichtlich verschiedener Tabletten- oder Tabletsparameter, wie Härte, Höhe oder Durchmesser und andere, gemessen und geprüft werden sollen. Die besondere Nützlichkeit des Verfahrens besteht darin, dass nunmehr auch die Messung des Durchmessers sowie die Durchführung eines Härtetests an einem Prüfling möglich ist, der wenigstens eine bombierte oder gewölbte Hauptoberfläche aufweist, beispielsweise linsenförmig gestaltet ist. Das Arbeitsverfahren bewirkt, dass der Prüfling spätestens bei der Anlage am Gegenbacken zur Bestimmung des Durchmessers oder zur Durchführung des Härtetests waagrecht liegt; ein Verkippen wenigstens zu diesem Zeitpunkt ist ausgeschlossen.

### Liste der Bezugszeichen

- 1, 25, 30: Stößel
- 2: beweglicher Backen oder Preßbacken
- 3, 21: feststehende Backen oder Gegenbacken
- 4: Schubfläche des Preßbackens
- 5, 22: Stirnwände der Backen 2, 3, 22
- 6, 34: gerade Führungsbahn des Backens 3, 21 für den Prüfling
- 7, 23: linsenförmige Tablette oder Dragee
- 8: Kraftmessdose
- 9: Führungsschiene
- 10: Schlitten
- 11: Mitnehmer
- 12,13: Zahnräder
- 14: Zahnriemen
- 15: Elektromotor Schrittmotor)
- 16: Platte
- 17: Halteteil
- 18: elektrischer Anschluß der Kraftmessdose
- 19: Schrauben
- 20: Traverse
- 24,27: Rüttler
- 26: Angriffsfläche unterhalb der geraden Fläche 6,31,34 des Backens
- 28: Elektromagnet
- 29: Rückstellfeder
- 31, 32, 33: Bewegungspfeile
- 35: Videokamera
- 36: Längsachse des Stößels

## Patentansprüche

1. Verfahren zum Ausrichten und Verschieben eines Prüflings (7), wie Tabletten, Pillen, Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozeßschrittes, wie Härtetest, mit dem Prüfling (7), unter Zuhilfenahme von zwei sich gegenüberstehenden und relativ zueinander beweglichen Backen (2,3,21), von denen der bewegliche Preßbacken (2) den Prüfling (7) auf einer Führungsbahn (6,34) auf den feststehenden Gegenbacken (3,21) bis zum mechanischen Kontakt des Prüflings (7) mit dem Gegenbacken (3,21) vorschiebt, um den weiteren Prozeßschritt mit dem Prüfling (7) einzuleiten,
**dadurch gekennzeichnet,**
**dass** unter Mitnahme des Prüflings (7) die Vorschubbewegung des beweglichen Preßbackens (2) in Richtung des feststehenden Gegenbackens (3,21) fortlaufend unterbrochen und der Preßbacken (2) zurückgezogen und vorgeschoben wird, wobei die Vorschub- und Rückschubbewegung des Preßbackens (2) klein ist in Bezug auf seinen gesamten Vorschubweg bezüglich des Gegenbackens (3,21), insgesamt jedoch der Preßbacken (2) auf den Gegenbacken (3,21) unter Mitnahme des Prüflings (7) um jeweils die kleine Vorschubstrecke und Lösen von dem Prüfling mittels Rückschub zufährt, so dass der Prüfling in seine Ruhelage zurückschwingt und danach der Preßbacken (2) wieder zum Schub in Richtung des Gegenbackens (3,21) ansetzt, bis der Prüfling (7) den Gegenbacken (,21) erreicht hat
oder
der feststehende Backen (3,21) und/oder die Führungsbahn (6,34) wird während der Vorschubbewegung des beweglichen Preßbackens (2) in Richtung des feststehenden Gegenbackens (3,21) in Schwingungen versetzt, welche sich dem Prüfling (7) aufprägen, so dass derselbe während der Vorschubbewegung des Preßbackens (2) auch eine Kippschwingung und/oder Vibrationsschwingung durchführt, die den Prüfling (7) fortwährend in die Ruhelage zurück schwingen oder um dieselbe schwingen lässt, bis der Prüfling (7) den Gegenbacken (3,21) erreicht hat und die Schwingung des Prüflings (7) durch den feststehenden Gegenbacken (3,21) angehalten wird.

2. Verfahren nach Anspruch 1, **dadurch gekennzeichnet,**
**dass** die Vor- und Zurückbewegung des beweglichen Preßbackens (2) in Richtung des feststehenden Gegenbackens (3,21) so lange ausgeführt wird, bis der Prüfling (7) den feststehenden Gegenbacken (3,21) gerade berührt.

3. Verfahren nach Anspruch 1 oder 2, **dadurch gekennzeichnet,**
**dass** der bewegliche Preßbacken (2) den Prüfling (7) vor sich herschiebt und dabei den Prüfling (7) gegebenenfalls verkippt, bis derselbe an dem feststehenden Gegenbacken (3,21) anfährt, wonach der bewegliche Preßbacken (2) zurückgezogen wird, bis der Prüfling (7) in seine Ruhelage geschwungen ist und anschließend der Preßbacken (2) wiederum in Richtung des feststehenden Gegenbackens (3,21) zur Einleitung des Härtetests fährt.

4. Verfahren nach einem der vorstehenden Ansprüche,
**dadurch gekennzeichnet, dass** das Andocken des Prüflings (7) am feststehenden Gegenbacken (3,21) mittels einer Videokamera (35) überwacht wird, die beim Andocken ein Signal abgibt, welches zur Steuerung des Elektromotors (15) zum Antrieb des beweglichen Preßbackens (2) dient.

5. Verfahren nach Anspruch 1, **dadurch gekennzeichnet,**
**dass** der bewegliche Preßbacken (2) während seiner Vorschubbewegung in Richtung des feststehenden Gegenbackens (3,21) Vibrationen in Richtung seiner Längsachse (36) hin und zurück bezüglich des Gegenbackens (3,21) ausführt, welche der Vorschubbewegung des Preßbackens (2) überlagert werden.

6. Vorrichtung zum Ausrichten und Verschieben eines Prüflings (7), wie Tabletten, Pillen oder Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozeßschrittes, wie Härtetest, mit dem Prüfling (7), mit zwei sich gegenüberstehenden und relativ zueinander beweglichen Backen (2,3,21), von denen der bewegliche Preßbacken (2) den Prüfling (7) auf einer Führungsbahn (6,34) auf den feststehenden Gegenbacken (3,21) bis zum mechanischen Kontakt des Prüflings (7) mit dem Gegenbacken (3,21) zur Einleitung des weiteren Prozeßschritts mit dem Prüfling (7) zuschiebt,
**dadurch gekennzeichnet,**
**dass** der Preßbacken (2) unter fortlaufender Unterbrechung seiner Vorschubbewegung kleine Vorschub- und Rückschubstrecken in Bezug auf seinen gesamten Vorschubweg vor und zurück bezüglich des Gegenbackens (3,21) verfahrbar ist, insgesamt jedoch auf den Gegenbacken (3,21) unter Mitnahme des Prüflings (7) um jeweils die kleine Vorschubstrecke und Lösen von dem Prüfling mittels Rückschub, bis der Prüfling (7) den Gegenbacken erreicht (3,21), zuzufahren imstande ist
oder
**dass** der feststehende Backen (3,21) und/oder die Führungsbahn (6,34) während der Vorschubbewegung des beweglichen Preßbackens (2) in Richtung auf den feststehenden Gegenbacken (3,21) um eine Ruhelage mittels eines Schwingungserzeugers (24,27) in Schwingungen versetzbar ist, welche sich dem Prüfling (7) aufprägen, so dass derselbe während der Vorschubbewegung auch eine Kippschwingung bzw. Vibrationsschwingung durchzuführen imstande ist.

7. Vorrichtung nach Anspruch 6, **dadurch gekennzeichnet,**
**dass** der bewegliche Preßbacken (2) sowie eine daran befestigte Kraftmeßdose (8) zur Bildung eines Härtetesters auf einem Schlitten (10) angeordnet sind, welcher auf einer feststehenden Führungsschiene (9) verfahrbar ist und welcher mittels eines elektrischen Schrittmotors (5) sowie eines Antriebsvorgeleges (12,13,14) um kleine Vorschub- und Rückschubstrecken in Bezug auf seinen gesamten Vorschubweg vor und zurück bezüglich des Gegenbackens (3,21) verfahrbar ist.

8. Vorrichtung nach Anspruch 7, **dadurch gekennzeichnet,**
**dass** das Antriebsvorgelege ein Zahnradvorgelege (12,13) ist und aus zwei Zahnrädern (12,13) besteht, welche über einen Zahnriemen (14) miteinander verbunden sind.

9. Vorrichtung nach Anspruch 6, **dadurch gekennzeichnet, dass** der Schwingungserzeuger zur Erzeugung einer Vibration des feststehenden Gegenbackens (3,21) und/oder der Führungsbahn (6,34) des Prüflings (7) ein Rüttler (24,27) ist, welcher einen Stößel (30) aufweist, der an dem feststehenden Gegenbacken (3,21) und/oder der Führungsbahn (6,34) angreift.

10. Vorrichtung nach Anspruch 9, **dadurch gekennzeichnet, dass** der Rüttler (24,27) einen Elektromagneten (28) sowie eine Rückstellfeder (29) aufweist.

11. Vorrichtung nach einem der Ansprüche 7 bis 10,
**dadurch gekennzeichnet, dass** oberhalb des feststehenden Gegenbackens (3,21) eine Videokamera (35) zum Überwachen des Andockens des Prüflings (7) an dem Gegenbacken (3,21) angeordnet ist, welche ein elektrisches Signal erzeugt, welches zur Beeinflussung des Elektromotors (15) dient.

12. Vorrichtung zum Ausrichten und Verschieben eines Prüflings (7), wie Tabletten, Pillen oder Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozeßschrittes, wie Härtetest oder Überschub, mit dem Prüfling (7), mit einem beweglichen Schubbacken (2) zur Bewegung des Prüflings (7) auf einer Führungsbahn (6,34) zur Einleitung des weiteren Prozeßschritts mit dem Prüfling (7), **dadurch gekennzeichnet,**
**dass** der Schubbacken (2) unter fortlaufender Unterbrechung seiner Vorschubbewegung kleine Vorschub- und Rückschubstrecken in Bezug auf seinen gesamten Vorschubweg vor und zurück verfahrbar ist, insgesamt jedoch unter Mitnahme des Prüflings (7) auf der Führungsbahn (6,34) um jeweils die kleine Vorschubstrecke und Lösen von dem Prüfling mittels Rückschub, bis der Prüfling (7) den gewünschten Ort erreicht (3,21), zu verfahren imstande ist oder dass die Führungsbahn (6,34) während der Vorschubbewegung des beweglichen Schubbackens (2) um eine Ruhelage mittels eines Schwingungserzeugers (24,27) in Schwingungen versetzbar ist, welche sich dem Prüfling (7) aufprägen, so dass derselbe während der Vorschubbewegung des Schubbackens (2) auch eine Kippschwingung bzw. Vibrationsschwingung durchzuführen imstande ist.

## Claims

1. A method for aligning and moving a test specimen (7) such as, for instance, tablets, pills, lozenges or capsules, which are cambered or rounded on at least one of their main surfaces and are thus able to execute rocking oscillations starting from a resting position, in order to carry out another process step such as a hardness test, involving the test specimen (7), making use of two jaws (2, 3, 21) arranged opposite from each other and movably relative to each other, whereby the movable pressing jaw (2) pushes the test specimen (7) forward on a guide segment (6, 34) towards the stationary counter-jaw (3, 21) until the test specimen (7) makes mechanical contact with the counter-jaw (3, 21), so as to initiate the further process step involving the test specimen (7),
**characterized in that**,
while carrying along the test specimen (7), the advancing movement of the movable pressing jaw (2) in the direction of the stationary counter-jaw (3, 21) is continuously interrupted and the pressing jaw (2) retreats and advances, whereby the advancing and retreating movement of the pressing jaw (2) is small relative to its total advancing distance with respect to the counter-jaw (3, 21), but all in all, the pressing jaw (2) moves towards the counter-jaw (3, 21) while each time carrying along the test specimen (7) by the small advancing distance and releasing the test specimen by retreating, so that the test specimen oscillates back into its resting position and then the pressing jaw (2) starts up again to push in the direction of the counter-jaw (3, 21) until the test specimen (7) has reached the counter-jaw (3, 21)
or
the stationary jaw (3, 21) and/or the guide segment (6, 34) are made to oscillate during the advancing movement of the movable pressing jaw (2) in the direction of the stationary jaw (3, 21), said oscillations acting on the test specimen (7) so that, during the advancing movement of the pressing jaw (2), the test specimen (7) executes a rocking and/or vibrating oscillation that continuously causes the test specimen (7) to oscillate back into the resting position or allows it to oscillate around said resting position until the test specimen (7) has reached the counter-jaw (3, 21) and the oscillation of the test specimen (7) is stopped by the stationary counter-jaw (3, 21).

2. The method according to Claim 1, **characterized in that**
the forward and backward movement of the movable pressing jaw (2) in the direction of the stationary counter-jaw (3, 21) is carried out until the test specimen (7) is just touching the stationary counter-jaw (3, 21).

3. The method according to Claim 1 or 2, **characterized in that**
the movable pressing jaw (2) pushes the test specimen (7) along in front of it while, if applicable, tilting the test specimen (7) until the latter reaches the stationary counter-jaw (3, 21), after which the movable pressing jaw (2) retreats until the test specimen (7) has oscillated into its resting position, and subsequently the pressing jaw (2) moves once again in the direction of the stationary counter-jaw (3, 21) in order to initiate the hardness test.

4. The method according to one of the preceding claims,
**characterized in that** the docking of the test specimen (7) at the stationary counter-jaw (3, 21) is monitored by means of a video camera (35) that emits a signal at the time of the docking which serves to control the electric motor (15) that drives the movable pressing jaw (2).

5. The method according to Claim 1, **characterized in that**,
during its advancing movement in the direction of the stationary counter-jaw (3, 21), the movable pressing jaw (2) executes vibrations in the direction of its longitudinal axis (36) back and forth relative to the counter-jaw (3, 21), said vibrations being superimposed upon the advancing movement of the pressing jaw (2).

6. A device for aligning and moving a test specimen (7) such as, for instance, tablets, pills, lozenges or capsules, which are cambered or rounded on at least one of their main surfaces and are thus able to execute rocking oscillations starting from a resting position, in order to carry out another process step such as a hardness test, involving the test specimen (7), with two jaws (2, 3, 21) arranged opposite from each other and movably relative to each other, whereby the movable pressing jaw (2) pushes the test specimen (7) forward on a guide segment (6, 34) towards the stationary counter-jaw (3, 21) until the test specimen (7) makes mechanical contact with the counter-jaw (3, 21), so as to initiate the further process step involving the test specimen (7),
**characterized in that**,
the pressing jaw (2), with a continuous interruption of its advancing movement, can be moved back and forth along small advancing and retreating distances relative to its total advancing distance with respect to the counter-jaw (3, 21), but all in all, it is able to move towards the counter-jaw (3, 21) while carrying along the test specimen (7) by the small advancing distance and releasing the test specimen by retreating, until the test specimen (7) has reached the counter-jaw (3, 21)
or
the stationary jaw (3, 21) and/or the guide segment (6, 34) can be made to oscillate around a resting position during the advancing movement of the movable pressing jaw (2) in the direction of the stationary jaw (3, 21) by means of a oscillation generator (24, 27), said oscillations acting on the test specimen (7) so that, during the advancing movement of the test specimen (7), it is able to execute a rocking and/or vibration oscillation.

7. The device according to Claim 6, **characterized in that**
the movable pressing jaw (2) as well as a load cell (8) attached to it to create a hardness tester are arranged on a carriage (10) that can be moved on a stationary guide rail (9) and that can be moved back and forth by means of an electric stepping motor (5) as well as by a drive gear (12, 13, 14) by small advancing and retreating distances relative to its total advancing distance with respect to the counter-jaw (3, 21).

8. The device according to Claim 7, **characterized in that**
the drive gear is a toothed gear (12, 13) and consists of two toothed wheels (12, 13) which are connected to each other via a toothed segment (14).

9. The device according to Claim 6, **characterized in that** the oscillation generator for generating a vibration of the stationary counter-jaw (3, 21) and/or of the guide segment (6, 34) of the test specimen (7) is a vibrator (24, 27) that has a plunger (30) that engages the stationary counter-jaw (3, 21) and/or the guide segment (6, 34).

10. The device according to Claim 9, **characterized in that** the vibrator (24, 27) has an electromagnet (28) as well as a pull-back spring (29).

11. The device according to one of Claims 7 to 10,
**characterized in that**, above the stationary counter-jaw (3, 21), there is a video camera (35) for monitoring the docking of the test specimen (7) at the stationary counter-jaw (3, 21), said camera generating an electric signal that serves to influence the electric motor (15).

12. A device for aligning and moving a test specimen (7) such as, for instance, tablets, pills, lozenges or capsules, which are cambered or rounded on at least one of their main surfaces and are thus able to execute rocking oscillations starting from a resting position, in order to carry out another process step such as a hardness test or pushing over, involving the test specimen (7), having a movable pushing jaw (2) for moving the test specimen (7) on a guide segment (6, 34) so as to initiate the further process step involving the test specimen (7), **characterized in that**, the pushing jaw (2), with a continuous interruption of its advancing movement, can be moved back and forth along small advancing and retreating distances relative to its total advancing distance, but all in all, while each time carrying along the test specimen (7) on the guide segment (6, 34) by the small advancing distance and releasing the test specimen by retreating, can be moved until the test specimen (7) has reached the desired place (3, 21), or **in that** the guide segment (6, 34) can be made to oscillate around a resting position during the advancing movement of the movable pushing jaw (2) by means of an oscillation generator (24, 27), said oscillations acting on the test specimen (7) so that the test specimen (7) is also able to execute a rocking and/or vibration oscillation during the advancing movement of the movable pressing jaw (2).

## Revendications

1. Procédé pour orienter et déplacer une éprouvette (7), par exemple des comprimés, pilules, dragées ou pastilles, laquelle est bombée ou arrondie sur au moins l'une de ses surfaces principales et est ainsi en mesure d'effectuer des oscillations basculantes partant d'une position de repos, pour exécuter une autre étape de process avec l'éprouvette (7), telle qu'un test de dureté, en ayant recours à deux mâchoires (2, 3, 21) qui se font face et qui sont mobiles l'une par rapport à l'autre, desquelles la mâchoire de serrage mobile (2) pousse l'éprouvette (7) sur une glissière de guidage (6, 34) vers la contre-mâchoire fixe (3, 21) jusqu'au contact mécanique de l'éprouvette (7) avec la contre-mâchoire (3, 21), pour lancer l'autre étape de process avec l'éprouvette (7),
**caractérisé en ce que**
tout en entraînant l'éprouvette (7), le mouvement d'avance de la mâchoire de serrage mobile (2) en direction de la contre-mâchoire fixe (3, 21) est interrompu de façon continue et la mâchoire de serrage (2) recule et avance, le mouvement d'avance et de recul de la mâchoire de serrage (2) étant petit au vu de la course totale par rapport à la contre-mâchoire (3, 21), dans l'ensemble cependant, la mâchoire de serrage (2) se dirige vers la contre-mâchoire (3, 21) entraînant l'éprouvette (7) à chaque fois en parcourant le petit trajet vers l'avant et se séparant de l'éprouvette (7) moyennant un mouvement de recul, de sorte que l'éprouvette bascule dans sa position de repos et qu'ensuite la mâchoire de serrage (2) entame à nouveau une poussée en direction de la contre-mâchoire (3, 21), jusqu'à ce que l'éprouvette (7) atteigne la contre-mâchoire (3, 21)
ou
la mâchoire fixe (3, 21) et/ou la glissière de guidage (6, 34) se mettent à vibrer, pendant le mouvement d'avance de la mâchoire de serrage mobile (2) en direction de la contre-mâchoire fixe (3, 21), animant l'éprouvette (7), de sorte que celle-ci effectue pendant le mouvement d'avance de la mâchoire de serrage (2) également des oscillations basculantes et/ou des oscillations vibratoires qui ramènent constamment l'éprouvette (7) dans sa position de repos ou la font osciller autour de celle-ci, jusqu'à ce que l'éprouvette (7) ait atteint la contre-mâchoire (3, 21) et que l'oscillation de l'éprouvette (7) soit arrêtée par la contre-mâchoire fixe (3, 21).

2. Procédé selon la revendication 1, **caractérisé en ce que**
le mouvement d'avance et de recul de la mâchoire de serrage mobile (2) en direction de la contre-mâchoire fixe (3, 21) est effectué jusqu'à ce que l'éprouvette (7) entre tout juste en contact avec la contre-mâchoire (3, 21).

3. Procédé selon la revendication 1 ou 2, **caractérisé en ce que**
la mâchoire de serrage mobile (2) pousse l'éprouvette (7) devant soi faisant basculer le cas échéant l'éprouvette (7) jusqu'à ce que celle-ci arrive contre la contre-mâchoire fixe (3, 21), ce après quoi la mâchoire de serrage mobile (2) se retire jusqu'à ce que l'éprouvette (7) oscille jusque dans sa position de repos et ensuite la mâchoire de serrage (2) part à nouveau en direction de la contre-mâchoire fixe (3, 21) pour lancer le test de dureté.

4. Procédé selon l'une quelconque des revendications ci-avant,
**caractérisé en ce que**
l'arrivée de l'éprouvette (7) contre la contre-mâchoire fixe (3, 21) est surveillée par une caméra vidéo (35) qui émet lors du premier contact d'arrivée un signal servant à la commande du moteur électrique (15) pour l'entraînement de la mâchoire de serrage mobile (2).

5. Procédé selon la revendication 1, **caractérisé en ce que**
la mâchoire de serrage mobile (2) effectue au cours de son mouvement d'avance en direction de la contre-mâchoire fixe (3, 21) des vibrations dans le sens de son axe longitudinal (36), en avant et en arrière par rapport à la contre-mâchoire (3, 21), lesquelles sont superposées au mouvement d'avance de la mâchoire de serrage (2).

6. Dispositif pour orienter et déplacer une éprouvette (7), par exemple des comprimés, pilules, dragées ou pastilles, laquelle est bombée ou arrondie sur au moins l'une de ses surfaces principales et est ainsi en mesure d'effectuer des oscillations basculantes partant d'une position de repos, pour exécuter une autre étape de process avec l'éprouvette (7), telle qu'un test de dureté, en ayant recours à deux mâchoires (2, 3, 21) qui se font face et qui sont mobiles l'une par rapport à l'autre, desquelles la mâchoire de serrage mobile (2) pousse l'éprouvette (7) sur une glissière de guidage (6, 34) vers la contre-mâchoire fixe (3, 21) jusqu'au contact mécanique de l'éprouvette (7) avec la contre-mâchoire (3, 21), pour lancer l'autre étape de process avec l'éprouvette (7),
**caractérisé en ce que**
la mâchoire de serrage (2) est déplaçable tout en subissant de façon continue une interruption de son mouvement d'avance sur de petits trajets en avant et en arrière par rapport à sa course totale en avant et en arrière vis-à-vis de la contre-mâchoire (3, 21), dans l'ensemble cependant est en mesure d'avancer vers la contre-mâchoire (3, 21) en entraînant l'éprouvette (7) à chaque fois sur le petit trajet en avant et se séparant de l'éprouvette moyennant un mouvement en arrière, jusqu'à ce que l'éprouvette (7) atteigne la contre-mâchoire (3, 21)
ou
pendant le mouvement d'avance de la mâchoire de serrage mobile (2) en direction de la contre-mâchoire fixe (3, 21), la mâchoire fixe (3, 21) et/ou la glissière de guidage (6, 34) peuvent se mettre à vibrer autour d'une position de repos moyennant un générateur de vibrations (24, 27), animant l'éprouvette (7), de sorte que celle-ci est aussi en mesure d'effectuer pendant le mouvement d'avance des oscillations basculantes ou des oscillations vibratoires.

7. Dispositif selon la revendication 6, **caractérisé en ce que**
la mâchoire de serrage mobile (2), ainsi qu'une boîte dynanométrique (8) qui y est fixée pour former un testeur de dureté sont disposés sur un chariot (10) qui est déplaçable sur un rail de guidage fixe (9) et qui est déplaçable au moyen d'un moteur électrique pas à pas (5), ainsi que d'une transmission de commande (12, 13, 14) sur des trajets d'avance et de recul petits par rapport à la course totale en avant et en arrière vis-à-vis de la contre-mâchoire (3, 21).

8. Dispositif selon la revendication 7, **caractérisé en ce que**
la transmission de commande est un train d'engrenage (12, 13) constitué par deux roues dentées (12, 13) reliées entre elles par une courroie dentée (14).

9. Dispositif selon la revendication 6, **caractérisé en ce que**
le générateur de vibrations destiné à produire une vibration de la contre-mâchoire fixe (3, 21) et/ou de la glissière de guidage (6, 34) de l'éprouvette (7) est un vibrateur (24, 27) qui présente un pilon (30) qui s'applique sur la contre-mâchoire fixe (3, 21) et/ou sur la glissière de guidage (6, 34).

10. Dispositif selon la revendication 9, **caractérisé en ce que** le vibrateur (24, 27) présente un électroaimant (28), ainsi qu'un ressort de rappel (29).

11. Dispositif selon l'une ou l'autre des revendications 7 à 10,
**caractérisé en ce que** au-dessus de la contre-mâchoire fixe (3, 21) est disposée une caméra vidéo (35) destinée à surveiller l'arrivée de l'éprouvette (7) contre la contre-mâchoire fixe (3, 21), laquelle caméra émet un signal électrique qui sert à influencer le moteur électrique (15).

12. Dispositif pour orienter et déplacer une éprouvette (7), par exemple des comprimés, pilules, dragées ou pastilles, laquelle est bombée ou arrondie sur au moins l'une de ses surfaces principales et est ainsi en mesure d'effectuer des oscillations basculantes partant d'une position de repos, pour exécuter une autre étape de process avec l'éprouvette (7), telle qu'un test de dureté ou la faire glisser, avec une mâchoire de poussée mobile (2) pour faire se mouvoir l'éprouvette (7) sur une glissière de guidage (6, 34) pour lancer l'autre étape de process avec l'éprouvette (7), **caractérisé en ce que**
la mâchoire de poussée (2) est déplaçable en étant interrompue de façon continue dans son mouvement d'avance, sur des trajets en avant et en arrière petits par rapport à sa course totale en avant et en arrière, dans l'ensemble cependant entraînant l'éprouvette (7) sur la glissière de guidage (6, 34) à chaque fois parcourant le petit trajet vers l'avant et se séparant de l'éprouvette moyennant un mouvement de recul, jusqu'à ce que l'éprouvette (7) ait atteint l'endroit souhaité (3, 21) ou que la glissière de guidage (6, 34) pendant le mouvement d'avance de la mâchoire de poussée (2) peut se mettre à vibrer autour d'une position de repos par l'action d'un générateur de vibrations (24, 27) lesquelles animent l'éprouvette (7) de sorte que celle-ci, pendant le mouvement d'avance de la mâchoire de poussée (2), est également en mesure d'effectuer une oscillation basculante ou une oscillation vibratoire.
